## Europäisches Patentamt
## European Patent Office
## Office européen des brevets

(11) Publication number: **0 165 657**
**B1**

(12) **EUROPEAN PATENT SPECIFICATION**

(45) Date of publication of patent specification: **12.07.89**

(51) Int. Cl.⁴: **A 61 F 9/00, A 61 B 17/32**

(21) Application number: **85301969.3**

(22) Date of filing: **21.03.85**

(54) **Knife for cataract surgery.**

(30) Priority: **23.03.84 US 592607**

(43) Date of publication of application:
**27.12.85 Bulletin 85/52**

(45) Publication of the grant of the patent:
**12.07.89 Bulletin 89/28**

(84) Designated Contracting States:
**BE CH DE FR GB IT LI NL SE**

(56) References cited:
**CH-A- 490 072**
**DE-A-3 205 959**
**US-A-1 775 813**
**US-A-2 951 482**
**US-A-3 670 733**
**US-A-3 759 263**
**US-A-3 886 656**
**US-A-3 913 585**
**US-A-4 011 870**
**US-A-4 180 075**
**US-A-4 184 491**
**US-A-4 445 517**

(73) Proprietor: **ALCON PHARMACEUTICALS LIMITED**
**P.O.Box 80**
**CH-6330 Cham (CH)**

(72) Inventor: **Schmidt, Friedrich W.**
**Ridge Avenue R.D. No. 4**
**Ephrata Pennsylvania 17522 (US)**

(74) Representative: **Bass, John Henton et al**
**REDDIE & GROSE 16 Theobalds Road**
**London WC1X 8PL (GB)**

Courier Press, Leamington Spa, England.

## Description

The present invention relates generally to surgical instruments and relates more particularly to a knife for use in ophthalmic surgery such as cataract surgery.

Ophthalmic surgery is inherently complicated by the difficulty in gaining access to the working area. Because of the overlying presence of the cornea, which must not be damaged, access to the interior of the eye and particularly the anterior capsule is usually gained indirectly through a small incision at the edge of the cornea where it joins the sclera.

In the surgical procedure known as an anterior capsulectomy, for which the present invention is particularly adapted, a substantial portion of the anterior lens capsule is removed. Since the capsule is a very thin, transparent, cellophane-like element, the cutting and removal of a portion thereof is a delicate procedure, especially in view of the attachment of the capsule to the zonules which comprise the suspensory ligaments of the lens. Damage to the zonules must be avoided so that the zonules can evenly support the remaining portion of the capsule that is left after performance of the capsulectomy. Furthermore, uncontrolled tearing or splitting of the anterior capsule can jeopardise the integrity of the posterior capsule with a resulting vitreous loss.

Various instruments have been used for the anterior capsulectomy procedure. At one time, capsule cutting forceps were the preferred instrument, but the lack of control of the cutting pattern and the danger of inadvertent tears through the posterior capsule or zonules were significant shortcomings. More recently, cutting cystitomes have been used, as have scissors, particularly with the microsurgical techniques which permit an accurate viewing and manipulation of the capsule. In present practice, the cystitome and particularly the hooked cystitime enjoy popularity.

The surgical techniques employed in carrying out the anterior capsulectomy are closely related to the capabilities of the instruments employed. Using a purposely dull cystitome, one conventional technique known as the "Christmas tree" technique, involves the tearing of the capsule to create an initial tree-like opening, following which a series of closely spaced small tears are employed to complete the procedure. This technique is somewhat unpredictable due to the very nature of the procedure and depends to a large extent on the skill of the surgeon. The tear may become excessively wide, endangering the zonules and requiring supplemental surgical procedures.

Another currently popular anterior capsulectomy technique is known as the can opener technique and is characterized by a series of small linked tears or punctures made by a sharp cystitome in the desired fragment pattern. Although the results are somewhat more controlled than the Christmas tree technique, there is still an undesirable lack of accuracy in the cutting pattern and a certain amount of tearing is involved both in the sequential puncturing strokes of the cystitome as well as in the removal of the fragment. This tearing can have adverse consequences to the zonules if excessive tension is applied to the capsule. Furthermore, the jagged edges of the remaining capsule can create complications in subsequent aspirating procedures.

The tearing or puncturing techniques currently employed in anterior capsulectomies can to a large extent be attributed to the lack of a surgical instrument which can reliably cut the anterior capsule along a desired cutting path without adversely affecting the zonules or the posterior capsule.

A knife for ophthalmic surgery comprising a knife blade pivotally mounted on a support is disclosed in US—A—4 180 075. The support in this device comprises two probes for holding the eye in a fixed position and the fixed pivot point enables the knife to be directed by the surgeon to make an arcuate incision. A general purpose knife having a blade mounted to pivot about the shaft axis, whereby the blade has a cutting edge offset from and directed towards the blade pivot axis to provide a castering action of the blade in response to changes in movement of the shaft is known from US—A—3 886 656.

It is accordingly a first object of the present invention to provide a knife for use in cataract surgery which can effect a continuous reliable cutting action through the anterior capsule regardless of the pattern of the cutting path.

A further object of the invention is to provide a knife as described which will minimise the tension applied to the capsule during the capsulectomy and accordingly avert damage to the zonules or the posterior capsule.

Another object of the invention is to provide a knife as described which is relatively easy to use, significantly shortens the cutting procedure, and which effects a clean, predictable cutting action.

A still further object of the invention is to provide a knife as described of a relatively simple construction which can be economically manufactured.

The present invention comprises a knife for ophthalmic surgery comprising a knife blade pivotally mounted on a support characterised in that the support comprises an elongated rigid shaft, and the knife blade is pivotally mounted on said shaft proximate one end thereof, the pivot axis of said blade extending substantially perpendicularly to said shaft, said blade having a cutting edge offset from and directed toward said blade pivot axis to provide a castering action of said blade in response to changes in direction of movement of said shaft end.

The shaft is preferably hollow to permit the passage of an irrigating fluid therethrough during the cutting procedure. The shaft handle may comprise a luerlock hub to permit its connection to a syringe or other device.

Thus, a knife having a hollow shaft enables irrigating fluids to be administered to the anterior chamber during the capsulectomy.

The invention will be described in more detail, by way of example, with reference to the accompanying drawings, in which:

Fig. 1 is a side elevational view of a surgical knife in accordance with the present invention;

Fig. 2 is a top plan view of the knife shown in Fig. 1, partially showing a syringe in dot-dash lines attached to the knife handle;

Fig. 3 is an end elevational view of the knife of Fig. 1;

Fig. 4 is an enlarged side elevational view partly broken away and in section of the outer end of the knife showing details of the knife blade and, in dot-dash lines, the manner in which the blade may swivel;

Fig. 5 is a view partly in section taken along line 5—5 of Fig. 4 and also showing in broken and in dot-dash lines two alternate positions of the swivel blade;

Fig. 6 is a side elevational sectional view of an eye shown undergoing a surgical procedure utilizing the knife of the present invention;

Fig. 7 is a view showing the insertion of the knife into an incision in the edge of the cornea;

Fig. 8 is a view similar to Fig. 7 showing the manner in which the castered knife blade may be moved to effect a circular cutting path of the blade;

Fig. 9 is a view similar to Figs. 7 and 8 showing the withdrawl of the knife from the eye;

Fig. 10 is an enlargement of the portion A of Fig. 8;

Fig. 11 is an enlargement of the portion B of Fig. 8; and

Fig. 12 is an enlarged view showing the blade cutting through eye tissue.

Description of the preferred embodiment

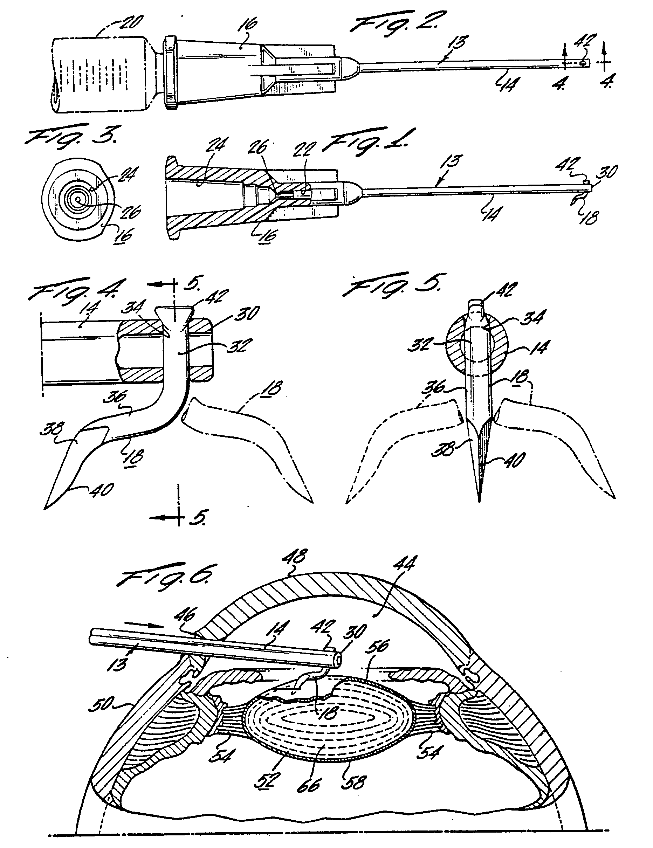

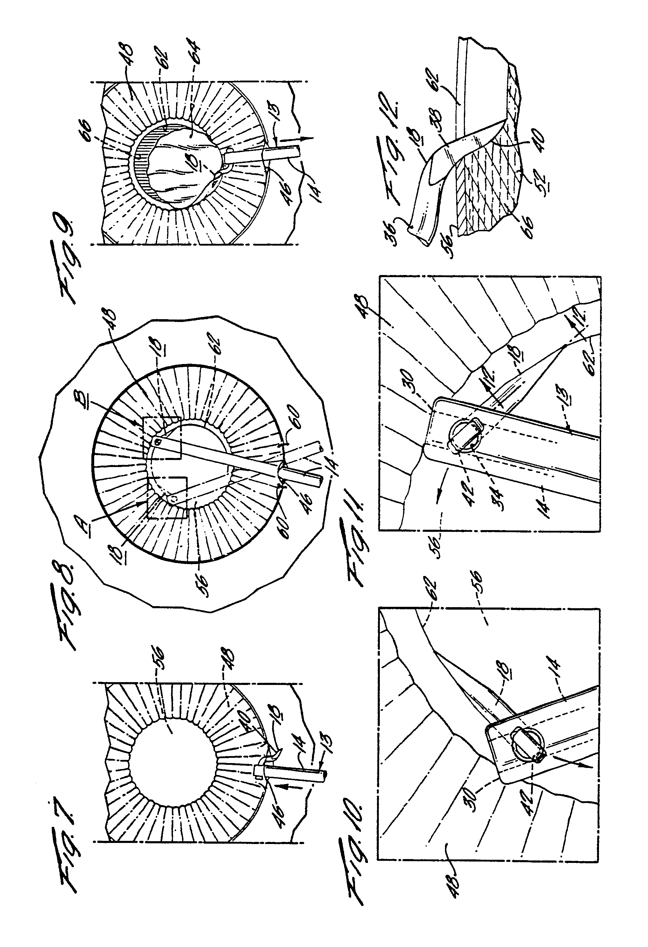

Referring to the drawings and particularly Fig. 1 thereof, a knife 13 in accordance with the present invention comprises a thin, hollow rigid shaft or cannula 14 having manual manipulating means 16 at one end and a knife blade 18 pivotally mounted proximate the other end thereof. The means 16 illustrated is in the form of a conventional luerlock hub to permit connection of a syringe 20 or other device as partially shown in dot-dash lines in Fig. 2. The shaft is secured within a bore 22 of the hub 16 and communicates with a tapered and stepped bore 24 opening outwardly at the opposite end of the hub. The bores 22 and 24 are connected by a restricted aperture 26. Fluid entering the bore 24, for example from the syringe 20, may accordingly pass through the hub and the shaft, issuing from the open distal end 30 of the shaft.

Although the means 16 could comprise a handle of various configurations, the preferred means is the illustrated conventional plastic hub commonly found associated with disposable hypodermic syringes. A reusable type of cannula having a metal hub could also be used.

The knife blade 18 as shown most clearly in the enlarged views of Figs. 4 and 5 comprises a cylindrical shank portion 32 pivotally disposed within a bore 34 in the shaft proximate the shaft distal end 30 and substantially perpendicular to the shaft axis. The blade 18 further includes an offset portion 36 extending away from the blade pivot axis and terminating in a point cutting portion 38 having a sharp cutting edge 40 directed toward the blade pivot axis. The cutting portion 38 is raked away from the pivot axis and the cutting edge is preferably double-beveled as shown in Fig. 5.

In the preferred embodiment illustrated, the blade 18 is retained within the bore 34 by the deformation of the extending end of the shank portion 32 to form a retaining head 42. The combination of the head 42 and the curve of offset portion 36 of the blade limits movement of the blade axially within the bore 34 of the shaft.

As shown in dot-dash lines in Fig. 4 and in dot-dash and in broken lines in Fig. 5, the blade 18 may freely swivel 360° within the bore 34 of the shaft. The offset in the blade as well as the direction of the cutting edge 40 toward the blade pivot axis provides a castering action of the blade during cutting movement of the knife.

The principal intended use of the present knife is illustrated in Fig. 6 which shows, in an enlarged sectional view, an anterior capsulectomy procedure in progress. The knife is introduced into the anterior chamber 44 of an eye through a small incision 46 in the region where the cornea 48 joins with the sclera 50. The lens 52 is supported by the suspensory ligaments 54 which are attached peripherally to the lens capsule comprised of the anterior capsule 56 and posterior capsule 58. The lens capsule is extremely thin and transparent and the cutting of the anterior capsule is a very delicate operation. Although operating microscopes have largely alleviated the difficulty in seeing the progress of the procedure, there still remain the dangers of inadvertently tearing the capsule beyond the intended area of the cut, the risk of damage to the suspensory ligaments, as well as the continuing concern with the inner surface of the cornea which must not be allowed to engage the cutting instrument. In this respect, it is important that the incision 46 be quite small and remain relatively sealed during the procedure to maintain the pressure in the anterior chamber 44 and thus prevent the collapse of the cornea. Fluid may be introduced through the hollow shaft 14 during the procedure to assist in maintaining the pressure in the anterior chamber.

The sequential steps of an anterior capsulectomy are illustrated in Figs. 7—9. In Fig. 7, the knife is shown being introduced through the incision 46. During introduction, the shaft 14 is rotated so that the pivot axis of the blade 18 lies generally in the plane of the incision. As the end of the knife is introduced into the incision, the knife pivots into the position illustrated in Fig. 7 with the cutting edge 40 trailing the advancing end of the shaft and the blade lying substantially in a common plane with the incision and the shaft.

When the shaft has been moved to approximately the twelve o'clock position within the eye, the shaft is rotated 90° about its longitudinal axis to position the blade cutting edge adjacent the anterior capsule of the lens. A slight downwardly directed force is imparted to the shaft to cause the cutting edge 40 of the blade to pierce the anterior capsule, and thus be positioned generally as illustrated in Fig. 12. Thereafter, the shaft is manipulated by the surgeon to cut the anterior capsule as hereafter described.

Temporary sutures may be provided at each end of the incision to partially close the incision and to minimize the loss of fluid pressure within the anterior chamber. Lateral movement of the knife shaft within the incision is thus quite limited although axial movement is not restricted. However, by virtue of the pivotally mounted knife blade and the offset position of the blade cutting edge directed toward the pivot axis, the blade responds to movement of the shaft distal end to provide a castering action of the blade. This castering action permits a continuous cutting motion of the knife in any selected path and allows the accurate controlled cutting of the anterior capsule to remove a capsule fragment of any desired shape. In the illustration of Fig. 8, the knife is being manipulated to produce a circular cut 62 in the anterior capsule, although any other desired cutting pattern could be chosen.

In Fig. 9, the withdrawal of the knife is illustrated, the sutures 60 having been removed just prior to this step. In Fig. 9, the cut fragment 64 of the anterior capsule is shown being withdrawn by the knife blade 18 although other means such as tissue forceps may be employed for this purpose. During the knife withdrawal, the shaft is rotated in a manner similar to that utilized during insertion of the knife so that the pivot axis of the knife blade will lie in the general plane of the incision. During withdrawal of the knife, the blade will pivot into the attitude shown in Fig. 9 with the blade cutting portion trailing behind and in the general plane of the shaft and incision whether or not the capsule fragment is withdrawn by the blade.

The enlarged views of Figs. 10 and 11 show the castering action of the blade in response to movements of the distal end of the shaft. It will be noted that in the case of an arcuate cut such as illustrated at 62 in both Figs. 10 and 11, the blade 18 is not aligned exactly tangentially with the cut since such an alignment would result in a decreasing cut radius and ultimately a straight cut. Instead, the alignment of the blade is slightly inwardly angled to enable the cutting edge to continue to follow the intended arcuate cutting path.

In Fig. 12, the blade cutting edge is shown cutting through the anterior capsule 56 with the tip of the blade penetrating into the lens cortex 66. As illustrated, the cutting edge should have a length which is considerably longer than the thickness of the capsule to insure a clean cutting action regardless of any deformations of the capsule which may take place given the jelly-like nature of the capsule support. The cutting edge should extend from the pointed distal end of the blade to the first bend in the blade to insure that the sharp cutting edge will be presented to the lens capsule at all times, regardless of the depth of penetration of the cutting portion of the blade. The raked angle of the cutting portion 38 of the blade as well as the convex curvature of the blade cutting edge combine to effect a slicing action of the blade which minimizes the possibility of tearing as opposed to the cutting of the capsule. The blade is preferably made of a heat treatable stainless steel wire stock by conventional techniques similar to those used for making cutting edged surgical needles.

As indicated above, the shaft 14 and manipulating means 16 in the preferred embodiment comprise a hubbed cannula of the kind conventionally used for disposable or reusable hypodermic needles. Such assemblies are widely available in a range of gauge sizes, and a preferred range for the present invention is between 20 gauge and 27 gauge. The conventionally available cannula are usually made of 300 series stainless steel.

Although it is not essential that the shaft 14 be hollow since the irrigation of the anterior chamber could be effected by auxiliary irrigating means, it is a desirable feature of the invention that by using a hollow shaft or cannula, fluids may be introduced into or removed from the anterior chamber during the surgical procedure without the complication of additional elements entering the chamber. As shown in Fig. 5, the shank portion 32 of the blade only partially obstructs the central shaft passage and fluids may pass therearound into the anterior chamber. The present instrument may thus by means of a quick disconnect type fluid fitting, such as a luerlock hub, be associated with a simple hypodermic syringe to provide irrigation during the surgical procedure to maintain the depth of the anterior chamber. Alternatively, the hollow shaft or cannula should be provided with a type of connection that mates with commercially available irrigation machines.

**Claims**

1. A knife for ophthalmic surgery comprising a knife blade (18) pivotally mounted on a support characterised in that the support comprises an elongated rigid shaft (14), and the knife blade is pivotally mounted on said shaft proximate one end thereof, the pivot axis of said blade extending substantially perpendicularly to said shaft, said blade having a cutting edge (40) offset from and directed toward said blade pivot axis to provide a castering action of said blade in response to changes in direction of movement of said shaft end.

2. The invention as claimed in claim 1 wherein said shaft (14) comprises a hollow shaft through which fluid may be administered to or removed from the cutting region.

3. A knife for ophthalmic surgery according to claim 2 including means (16) for manually manipulating said shaft (14) attached to one end of said shaft, passage means (22) in said manipulating means for introducing a fluid into or evacuating a fluid from said hollow shaft.

4. The invention as claimed in claim 3 wherein said manipulating means comprises a luerlock hub (16).

5. The invention as claimed in claim 1, 2 or 3 wherein said blade cutting edge (40) is raked at an acute angle with respect to said blade pivot axis.

6. A knife for ophthalmic surgery according to claim 4 wherein said blade comprises a substantially cylindrical shank portion (32) pivotally disposed within a bore (34) in said shaft (14), said blade being raked at an acute angle to said blade pivot axis.

7. The invention as claimed in any preceding claim wherein the distal end (38) of said blade (18) terminates in a sharp point.

## Patentansprüche

1. Messer zur Augenchirurgie mit einer an einem Träger drehbar gelagerten Messerklinge (18), dadurch gekennzeichnet, daß der Träger einen starren Schaft (14) von überwiegender Längserstreckung umfaßt, und die Messerklinge am Schaft in der Nähe eines Schaftendes drehbar gelagert ist, wobei die Drehachse der Klinge im wesentlichen rechtwinklig zum Schaft ist, die Klinge eine in bezug auf die Klingendrehachse versetzte und zu ihr hin gerichtete Schneidkante (40) hat, derart, daß bei Richtungsänderungen in der Beweung des Schaftendes ein Nachlaufverhalten der Klinge zustande kommt.

2. Erfindung nach Anspruch 1, bei der der Schaft (14) einen Hohlschaft umfaßt, durch den ein Fluid an die Schnittstelle zugeführt oder aus ihr entfernt werden kann.

3. Messer zur Augenchirurgie nach Anspruch 2, mit einer an einem Ende des Schaftes befestigten Vorrichtung (16) zum manuellen Handhaben des Schaftes (14), einem Durchlaß (22) in der Handhabungsvorrichtung zum Einführen oder Ableiten eines Fluides in bzw. aus dem Hohlschaft.

4. Erfindung nach Anspruch 3, bei der die Handhabungsvorrichtung eine Lüer-Look-Spritzenfassung umfaßt.

5. Erfindung nach Anspruch 1, 2 oder 3, bei der die Klingenschneidkante (40) unter einem spitzen Winkel zur Klingendrehachse angestellt ist.

6. Messer zur Augenchirurgie nach Anspruch 4, bei dem die Klinge einen im wesentlichen zylindrischen Schaftabschnitt (32) umfaßt, der in einer Bohrung (34) im Schaft (14) drehbar angeordnet ist, wobei die Klinge unter einem spitzen Winkel zur Klingendrehachse angestellt ist.

7. Erfindung nach einem der vorhergehenden Ansprüche, bei der das distale Endstück (38) der Klinge (18) in einer scharfen Spitze endet.

## Revendications

1. Couteau pour chirurgie ophtalmique comprenant une lame (18) de couteau montée en pivotement sur un support, caractérisé en ce que le support comprend un arbre rigide allongé (14), et la lame du couteau est montée en pivotement sur l'arbre à proximité de l'une de ses extrémités, l'axe de pivotement de la lame s'étendant pratiquement en étant perpendiculaire à l'arbre, la lame comprenant une arête coupante (40) décalée de l'axe de pivotement de la lame, et dirigée vers cet axe, afin de conférer une action de roulette à la lame en réponse à des changements du sens de déplacement de l'extrémité de l'arbre.

2. Invention selon la revendication 1, dans laquelle l'arbre (14) comprend un arbre creux par l'intermédiaire duquel un fluide peut être administré à la zone de coupe ou être enlevé de cette zone.

3. Couteau pour chirurgie ophtalmique selon la revendication 2, comprenant un moyen (16) pour manipuler manuellement l'arbre (14) fixé à une extrémité de l'arbre, un moyen de passage (22) dans le moyen de manipulation pour introduire un fluide dans l'arbre creux, ou l'évacuer de cet arbre.

4. Invention selon la revendication 3, dans laquelle le moyen de manipulation comprend un moyeu luerlock (16).

5. Invention selon la revendication 1, 2 ou 3, dans laquelle l'arête coupante (40) du couteau est inclinée suivant un angle aigu avec l'axe de pivotement de la lame.

6. Couteau pour chirurgie ophtalmique selon la revendication 4, dans lequel la lame comprend une partie de queue (32) sensiblement cylindrique disposée en pivotement à l'intérieure d'un alésage (34) de l'arbre (14), la lame étant inclinée suivant un angle aigu par rapport à l'axe de pivotement de la lame.

7. Invention selon l'une quelconque des revendications précédentes, dans laquelle l'extrémité distale (38) de la lame (18) se termine par une pointe aiguisée.

Fig. 2.

Fig. 3.

Fig. 1.

Fig. 4.

Fig. 5.

Fig. 6.

EP  0 165 657  B1

Fig. 9.

Fig. 12.

Fig. 8.

Fig. 11.

Fig. 7.

Fig. 10.

2